# EUROPEAN PATENT APPLICATION

(11) **EP 2 807 986 A1**
(43) Date of publication of application: **03.12.2014**
(21) Application number: 13740514.8
(22) Date of filing: 23.01.2013
(51) Int. Cl.: A61B 18/02, F25D 3/10

(54) **METHOD FOR FEEDING A CRYOGENIC AGENT TO A CRYOGENIC INSTRUMENT AND CRYOSURGICAL APPARATUS FOR IMPLEMENTING SAME**

(30) Priority: 26.01.2012 EA 201200066; 26.01.2012 EA 201200067
(71) Applicant: Semenov, Vyacheslav Yuryevich, Moscow 115162 (RU)
(72) Inventor: SEMENOV, Vyacheslav Yuryevich, Moscow 111562 (RU); SEMENOVA, Olga Pavlovna, Moscow 119021 (RU); MALININ, Nikolai Nikolaevich, St.Petersburg 196158 (RU)
(74) Representative: Delamare, Raoul
(86) International application number: PCT/EA2013/000001
(87) International publication number: WO 2013/110283

(57) **Abstract**

The invention relates to the field of medicine, specifically to methods for feeding a cryogenic agent to a cryogenic instrument, and also to cryosurgical apparatuses.

In accordance with the invention, additionally one forms a loop-shaped main line going out and entering into the reservoir with the cryogenic agent, realizes a circulation of the cryogenic agent into the loop-shaped main line, connects the cryogenic instrument to said loop-shaped main line via the cryogenic conduit and feeds through it the cryogenic agent, wherein a flow rate of the cryogenic agent circulated into the loop-shaped main line must be greater than a flow rate of the cryogenic agent entering into the cryogenic instrument via the cryogenic conduit. A cryosurgical apparatus is used to implement said method.

The technical result of the proposed solution is an increase in the distance over which it is possible to feed the cryogenic agent to the cryogenic instrument from the reservoir with the cryogenic agent while keeping the cryogenic instrument at a low temperature, and an increase in the operating efficiency of the cryosurgical apparatus, namely: the possibility of achieving temperatures as low as -200 and -210°C when using liquid nitrogen as the cryogenic agent and with a significant distance between the cryogenic instrument and the reservoir with the cryogenic agent.

## Description

### Field of the invention

The invention relates to the field of medicine, specifically to methods for feeding a cryogenic agent to cryogenic instruments as well as to a cryosurgical equipment, and can be used for cooling and destruction of pathologically changed areas of biological tissues.

### Background of the method

According to the first of its aspects, the invention relates to methods for feeding a cryogenic agent to a cryogenic instrument.

The prior art comprises a variety of methods for feeding a cryogenic agent to a cryogenic instrument. It is known a method for feeding a cryogenic agent to a cryogenic instrument which includes connecting of the cryogenic instrument to a reservoir with a cryogenic agent via a cryogenic conduit, wherein the cryogenic agent is fed from the reservoir to the cryogenic instrument via the cryogenic conduit, for this purpose the cryogenic agent is fed to the cryogenic instrument under pressure. (See description to the USSR patent No 1102096, published on January 11, 1983)

The drawback of the above method is that it does not allow for keeping a long distance between the cryogenic instrument and the reservoir with the cryogenic agent while maintaining low temperature, due to the fact that the more is the pressure in the cryogenic conduit required to feed the cryogenic agent on a longer distance, the higher is the boiling point of cryogenic agent. The minimum temperature that can be maintained using the above method for feeding the cryogenic agent with the help of liquid nitrogen is - 185°C.

Another method is described in the Russian patent No 2053719, published in 1996. This method involves feeding a cryogenic agent to a cryogenic instrument by using a vacuum pump-based exhaust system to generate vacuum in the cryogenic instrument itself. Such method allows lowering the cryogenic agent boiling point in the cryogenic instrument (up to - 210°C for liquid nitrogen).

The above method is the most similar to the solution claimed and is chosen herein as its prototype.

The drawback of the above method is that it does not allow for keeping a long distance between the cryogenic instrument and the reservoir with the cryogenic agent. This limits the scope of application of the cryosurgical apparatus and reduces its operating efficiency. This is attributable to the fact that the cryogenic agent starts boiling inside the cryogenic conduit, if the length of the latter is increased. Such phenomenon ultimately reduces the operating efficiency as it prevents attaining low temperatures inside the cryogenic instrument and makes the cooling of the same less efficient. However, a shorter cryogenic conduit means a more difficult manipulation of the cryogenic instrument during surgeries.

### Disclosure of the invention as a method

The invention relates to the field of medicine, specifically to methods for feeding a cryogenic agent to a cryogenic instrument, wherein one connects the cryogenic instrument to a reservoir with the cryogenic agent via a cryogenic conduit and feeds the cryogenic agent from the reservoir to the cryogenic instrument via the cryogenic conduit.

The claimed invention has the following technical objective, i.e. to increase the distance over which it is possible to feed the cryogenic agent to the cryogenic instrument from the reservoir with the cryogenic agent while keeping the cryogenic instrument at a low temperature.

The invention is described herein with the primary aim to offer a method for feeding a cryogenic agent to a cryogenic instrument that serves to mitigate the above drawback. For this purpose, the method for feeding a cryogenic agent to a cryogenic instrument as described in the above introduction to the general description essentially characterized in that additionally one forms a loop-shaped main line going out and entering into the reservoir with the cryogenic agent, realizes a circulation of the cryogenic agent into the loop-shaped main line, connects the cryogenic instrument to said loop-shaped main line via the cryogenic conduit and feeds through it the cryogenic agent, wherein a flow rate of the cryogenic agent circulated into the loop-shaped main line must be greater than a flow rate of the cryogenic agent entering into the cryogenic instrument via the cryogenic conduit.

Such method for feeding the cryogenic agent to the cryogenic instrument according to the invention makes it possible to increase the distance over which it is possible to feed the cryogenic agent to the cryogenic instrument from the reservoir with the cryogenic agent while keeping the cryogenic instrument at a low temperature.

There is a version of the above invention in that said feeding of the cryogenic agent from the loop-shaped main line to the cryogenic instrument is realized by generating an underpressure in the cryogenic instrument.

The above feature serves to increase cooling capacity of the cryogenic instrument and, as a result, tissue freezing rate during cryosurgeries. This is explained by the fact that in vacuum engineering a cryogenic agent flow through the cryogenic conduit is determined by the capacity of the pipeline which depends on the pressure difference at the ends of the cryogenic conduit and is in inverse ratio to the length of the cryogenic conduit. The pressure difference when operating in underpressure conditions cannot, by definition, be more than 1 atm. Therefore, if we reduce the length of a cryogenic conduit section operating in underpressure conditions (i.e. by using a loop-shaped main line) from 2 to 0,2 meters, we will be able to increase its capacity tenfold.

There is a version of the above invention in that said feeding of the cryogenic agent from the loop-shaped main line to the cryogenic instrument by generating a pressure in the cryogenic conduit connecting the loop-shaped main line and the cryogenic instrument.

The above feature allows to use a variety of cryogenic instruments: closed-type (where the cryogenic agent does not come into direct contact with the tissues of the patient under surgery) and open-type (where the cryogenic agent comes into direct contact with the tissues), and a spray nozzle with free discharge of the cryogenic agent from the cryogenic instrument for cryolavage of the patient tissues.

### Background of the device

According to the second of its aspects, the invention relates to cryosurgical apparatuses including a reservoir with a cryogenic agent, a cryogenic instrument with a feeding cryoconduit of the cryogenic agent and a draining cryoconduit of the cryogenic agent, an exhaust system of the cryogenic agent connected to the cryogenic instrument via the draining cryoconduit of the cryogenic agent.

The prior art comprises cryogenic instruments and apparatuses for local tissue freezing. A known example of the prior art is a cryosurgical apparatuses including a reservoir with a cryogenic agent, a cryogenic instrument with a feeding cryoconduit of the cryogenic agent and a draining cryoconduit of the cryogenic agent (See the USSR patent No 1102096, published on January 11, 1983), wherein cryogenic agent is fed to the cryogenic instrument under pressurized gas.

The drawback of the above method is that it does not allow to attain low temperature due to the fact that the more is the pressure, the higher is the boiling point of cryogenic agent. If liquid nitrogen is used as cryogenic agent, the minimum temperature delivered by said apparatus is -185 °C.

Also, a similar device is described in the Russian patent No 2053719, published in 1996. According to the state of the art, the cryogenic agent is fed to the cryogenic instrument by using a vacuum pump-based exhaust system to generate vacuum in the cryogenic instrument itself.

The above method is the most similar to the solution claimed and is chosen herein as its prototype.

The drawback of the above method is that it does not allow for keeping a long distance between the cryogenic instrument and the reservoir with the cryogenic agent. This limits the scope of the cryosurgical apparatus and reduces its operating efficiency. This is attributable to the fact that the cryogenic agent starts boiling inside the cryogenic conduit, if the length of the latter is increased, which ultimately reduces operating efficiency. A shorter cryogenic conduit means a more difficult manipulation of the cryogenic instrument during surgeries.

### Disclosure of the invention as a device

The invention claimed has the following technical objective, i.e. to increase the operating efficiency of the cryosurgical apparatus, namely: the possibility of achieving temperatures as low as -200 and -210°C when using liquid nitrogen as cryogenic agent and with a significant distance between the cryogenic instrument and the cryogenic agent-containing reservoir.

The invention relies on this novel observation with the primary aim to offer a cryosurgical apparatus that serves to mitigate the above drawback. For this purpose, the cryosurgical apparatus as described in the above introduction to the general description essentially characterizes in that the cryosurgical apparatus has a loop-shaped main line where circulates the cryogenic agent, an inlet and an outlet of the loop-shaped main line being connected to the reservoir with the cryogenic agent. Said loop-shaped main line has at least one bend for connecting to the cryogenic instrument via the feeding cryoconduit of the cryogenic agent.

Such structure of the cryosurgical apparatus according to the invention is more efficient in use as it makes it possible to attain a lower temperature of cryogenic agent in the cryogenic instrument and to considerably increase the distance between the cryogenic instrument from the cryogenic agent-containing reservoir.

Another technical result of the invention is the option to use the cryosurgical apparatus in various modes, including one non-operating mode and five operating modes.

Said cryogenic apparatus allows to use a variety of cryogenic instruments: closed-type (where the cryogenic agent does not come into direct contact with the tissues of the patient under surgery) and open-type (where the cryogenic agent comes into direct contact with the tissues), for this purpose, open-type cryogenic instruments may involve free discharge of the cryogenic agent from the cryogenic instrument (if spray nozzle in cryolavage mode is used as a cryogenic instrument) or may come without such option (if the open-type cryogenic instrument is pressed to the tissues under treatment). The following is a list of modes of the cryosurgical apparatus:
1. The mode for preparation of the cryosurgical apparatus to operation.
2. The mode for feeding cryogenic agent to the cryogenic instrument by generating pressure in the feeding cryogenic conduit for closed-type cryogenic instruments.
3. Cryolavage mode - feeding cryogenic agent to the cryogenic instrument by generating pressure in the feeding cryogenic conduit if a spray nozzle with a free discharge of the cryogenic agent from the cryogenic instrument is used.
4. The mode for feeding cryogenic agent to the cryogenic instrument by generating underpressure in the cryogenic instrument for closed-type cryogenic instruments.
5. The mode for feeding cryogenic agent to the cryogenic instrument by generating underpressure in the cryogenic instrument for open-type cryogenic instruments pressed to the tissues under treatment.
6. Warming mode - warming of a closed-type cryogenic instrument for safe removal of the same from the tissue under freezing.

According to the invention, the cryosurgical apparatus has a loop-shaped main line which enters and exits the cryogenic agent-containing reservoir, circulating the cryogenic agent. Said main line has at least one bend to connect to the cryosurgical apparatus via the cryogenic agent feeding cryoconduit.

Inclusion of the loop-shaped main line in the structure makes it possible to maximize the distance between the cryogenic instrument and the reservoir with the cryogenic agent while keeping the cryogenic instrument at a low temperature at the same time.

There is a version of the above invention in that the inlet of the loop-shaped main line is connected to an outlet of an intake switch of a liquid or a gas phase of the cryogenic agent into the reservoir, while an inlet of the intake switch of the gas phase is connected to a section of the reservoir which contains the gas phase of the cryogenic agent and an inlet of the intake switch of the liquid phase is connected to a section of the reservoir which contains the liquid phase of the cryogenic agent.

The above feature ensures accelerated warming of the cryogenic instrument.

In this invention, the loop-shaped main line where circulates the cryogenic agent principally includes at least one feeding pump.

The above feature makes it possible to circulate the cryogenic agent in the loop-shaped main line in addition to feeding the same to the cryogenic instrument. Said feeding pump enables feeding the cryogenic agent to several cryogenic instruments connected to the loop-shaped main line.

Also, there is a version of the above invention in that the loop-shaped main line where circulates the cryogenic agent includes at least one first controller of a cryogenic agent flow rate.

The inclusion of the first controller of the flow rate makes it possible to select a most saving mode when connecting various open- or closed- type cryogenic instruments.

There is a version of the above invention in that the loop-shaped main line where circulates the cryogenic agent includes at least one controller of a cryogenic agent pressure.

The inclusion of the controller of the cryogenic agent pressure makes it possible to feed the cryogenic agent to the cryogenic instrument at required pressure in required operating modes of the cryogenic instrument.

Also, there is a version of the above invention in that the loop-shaped main line where circulates the cryogenic agent includes at least a sensor of the cryogenic agent pressure.

The inclusion of the first sensor of the cryogenic agent pressure makes it possible to add a system for control and regulation of the cryosurgical apparatus.

There is a version of the above invention in that the loop-shaped main line where circulates the cryogenic agent includes at least one sensor of a cryogenic agent liquid phase in the closed main line.

The above feature makes it possible to control the completion of preparation of the cryogenic apparatus for operation.

There is a version of the above invention in that the feeding cryoconduit of the cryogenic agent from the loop-shaped main line to the cryogenic instrument includes at least one clack valve of the cryoconduit.

The above feature makes it possible to prevent leakage of the gas phase from the cryogenic instrument to the loop-shaped main line or ambient air leaks.

There is a version of the above invention in that the feeding cryoconduit of the cryogenic agent from the loop-shaped main line to the cryogenic instrument includes at least one shutoff valve. Said valve has two positions: "open" and "closed". If the valve is in the "open" position, the cryogenic agent is fed to the cryogenic instrument. If the valve is in the "closed" position, the cryogenic agent is not fed to the cryogenic instrument.

The above feature makes it possible to prevent cryogenic agent leaks from the cryogenic conduit and the loop-shaped main line in the mode for preparation of the cryosurgical apparatus for operation and during cryogenic instrument change when the cryosurgical apparatus is in an operating mode.

There is a version of the above invention in that the feeding cryoconduit of the cryogenic agent from the loop-shaped main line to the cryogenic instrument includes at least one first heater of the cryogenic agent.

The inclusion of the first heater enables forced accelerated warming of the cryogenic instrument.

There is a version of the above invention in that the feeding cryoconduit of the cryogenic agent from the loop-shaped main line to the cryogenic instrument includes at least one first sensor of temperature of the cryogenic agent.

The inclusion of the first sensor of temperature of the cryogenic agent makes it possible to control and regulate the cryogenic apparatus in operating modes.

There is a version of the above invention in that the feeding cryoconduit of the cryogenic agent from the loop-shaped main line to the cryogenic instrument includes at least one plug connection.

The above feature enables connection of various cryogenic instruments.

### Brief description of the drawings

Other distinguishing features and advantages of the invention are readily apparent from the description below which includes but is not limited to the following features, with reference to the figure attached:
- Figure 1 is a layout view of a cryosurgical apparatus, a system for control and regulation is not shown.

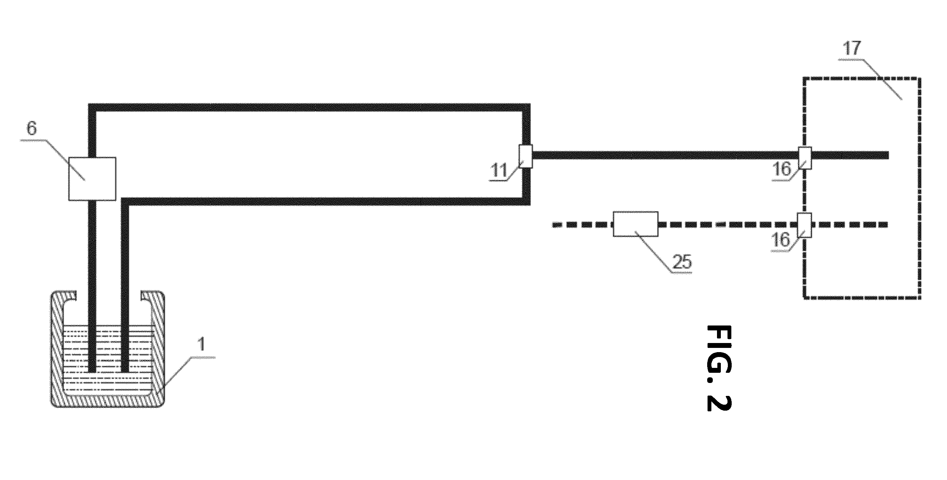

- Figure 2 shows a diagram of cryogenic agent feeding to the cryogenic instrument by generating underpressure.
- Figure 3 shows a diagram of feeding of a pressured cryogenic agent to the cryogenic instrument.
- Figure 4 is a sequence of actions for feeding the cryogenic agent to the cryogenic instrument.

A cryosurgical apparatus comprises a reservoir 1 with a cryogenic agent, a liquid cryogenic agent level indicator 2, a gas phase cryogenic agent intake 3, a liquid phase cryogenic agent intake 4, a valve 5, i.e. a switch of liquid-to-gas phase of the cryogenic agent, a feeding pump 6, a clack valve of the loop-shaped main line 7, a flow rate controller 8, a pressure sensor 9, a sensor 10 of the cryogenic agent liquid phase in the loop-shaped main line, a bend 11 for feeding the cryogenic agent from the loop-shaped main line to the cryogenic instrument, a clack valve 12 of the cryoconduit, a shutoff valve 13, a first cryogenic agent heater 14, a first temperature sensor 15 of the fed cryogenic agent, a plug connection 16, a cryogenic instrument 17 including a nozzle 18 spraying the cryogenic agent and a second temperature sensor 19 in the cryogenic instrument, a third temperature sensor in a draining cryoconduit 20, a second cryogenic agent heater 21, a vacuum valve 22, a vacuum sensor 23, a second (vacuum) flow rate controller 24, a vacuum pump 25, a silencer 26, a pressure controller 27 for the HI section of the loop-shaped main line.

The loop-shaped main line consists in sections: A (or A1), B, C, D, E, F, G, H, I.

The cryogenic conduit for feeding cryogenic agent to the cryogenic instrument consists in sections: K, L, M, N, O.

The cryogenic conduit for draining the gas-liquid phase of the cryogenic agent from the cryogenic instrument consists in sections: P, Q, R, S, T, U, V (or P, Q, R, S1).

Liquid nitrogen, liquid helium or other varieties of liquefied gas which remain in liquid state at normal air pressure can be used as cryogenic agent. A recuperator can be installed to reuse the cryogenic agent.

A standard Dewar flask can be used as reservoir 1.

Cryoconduits that may come in contact with the human body are equipped with reinforced thermal insulation (vacuum insulation, thermal blankets, etc), i.e. at any case they have at least two layers.

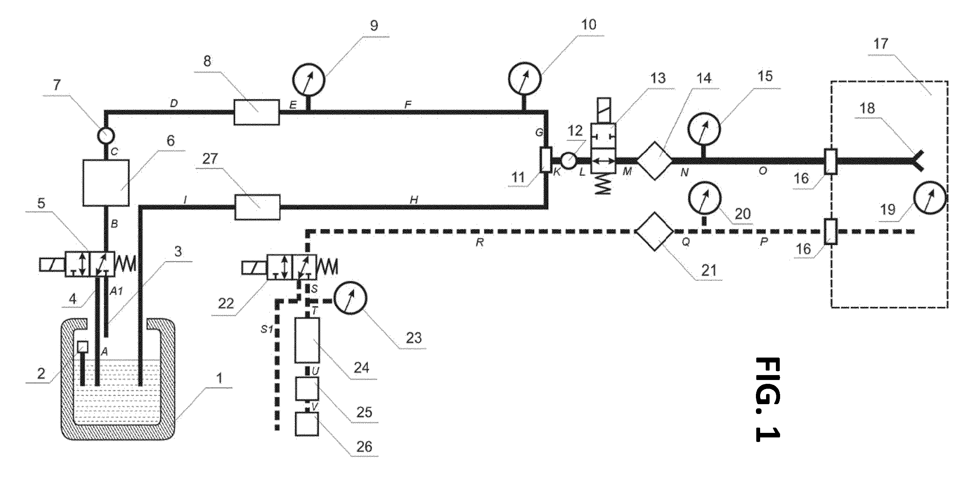

The feeding pump can be submerged (placed inside the reservoir 1 below the level of liquid cryogenic agent) or located outside the reservoir 1. Any type of the cryogenic feeding pump can be used, including positive displacement pump (gear pump, plunger pump, etc). The feeding pump can be adjustable or non-adjustable. If adjustable feeding pump is used (i.e. variable capacity pump), the first flow rate controller 8 is redundant. Figure 1 shows an option wherein a two-phase feeding pump 6 is used, i.e. capable of pumping cryogenic agent both in liquid and in gas states, for a more efficient warming. Figure 1 also shows an option with a non-adjustable feeding pump 6.

Sections A(or A1) BCDEFGHI of the main line, the feeding cryoconduit KLMNO and the gas-liquid phase draining cryoconduit PQRSTUV or PQRS1 from the cryogenic instrument may be integrated in a single insulated cryohose (including, in a coaxial way). This option is not shown in the Figure 1. Said option serves to improve the usability of the cryosurgical apparatus.

The control and regulation system serves to optimize the cryogenic apparatus operating modes to maintain minimum or predefined temperature and preset cryoexposure time by receiving the signals from the pressure sensor 9, the vacuum sensor 23, the first temperature sensor 15, the second temperature sensor 19, the third temperature sensor 20 and by controlling the operating modes of the feeding pump 6, the first flow rate controller 8, the second (vacuum) flow rate controller 24, the pressure controller 27, the cutoff valve 13, the vacuum valve 22, the first heater 14 and the second heater 21. The system is also connected to the liquid cryogenic agent level indicator 2.

The temperature inside the cryogenic instrument 17 or on its internal surface is measured by the temperature sensor 19. The temperature of cryogenic agent in the gas-liquid phase draining cryoconduit is measured by the temperature sensor 20, which transmits a signal to the control and regulation system. A signal from the second temperature sensor 19 is used to control the cryogenic instrument operating model, or the third temperature sensor 20, if a cryogenic instrument without a temperature sensor is used. This serves to make the operation more reliable and the cryosurgical apparatus control more accurate as well as to increase functional capabilities of the same.

The vacuum sensor 23 transmits a signal in accordance with the vacuum level in the draining cryogenic conduit to the control and regulation system. The second (vacuum) flow rate controller 24 sets a required mode to exhaust gas from the cryogenic instrument. The second heater 21 is designed to preheat the gas-liquid phase fed from the cryogenic instrument to protect the vacuum valve 22, the second (vacuum) flow rate controller 24 and the vacuum pump 25 from contact with liquid cryogenic agent.

The first flow rate controller 8 can be built as a controller which branches from the loop-shaped main line to drain cryogenic agent right to the reservoir 1. The element is not shown in the drawing.

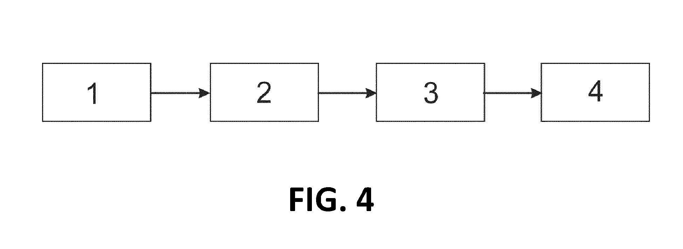

The Figure 4 demonstrates stages (by numbers) of the procedures for feeding the cryogenic agent to the cryogenic instrument, namely:
1. a loop-shaped main line which enters to and exits from the reservoir with the cryogenic agent is established;
2. the cryogenic agent is circulated in the loop-shaped main line;
3. the cryogenic instrument is connected to said loop-shaped main line via the cryogenic conduit;
4. cryogenic agent is fed to the cryogenic instrument via a cryogenic conduit.

### Embodiment of the invention.

The cryosurgical apparatus operates as follows.

In the mode 1, i.e. cryosurgical apparatus preparation for operation, the liquid cryogenic agent is fed from an external or a built-in reservoir 1 through an intake 4 using the feeding pump 6 of the liquid cryogenic agent to a loop-shaped main line ABCDEFGHI. For this purpose, the valve 5, i.e. gas/liquid phase intake switch, is in the liquid phase intake position (see Figure 1). The liquid cryogenic agent level indicator 2 sends a signal corresponding to the cryogenic agent level in the reservoir to the control and regulation system of the cryogenic apparatus. The cryogenic agent is fed to the bend 11 through the first flow rate controller 8, is circulated along the loop-shaped main line through the pressure controller 27, and fed back to the reservoir 1. The pressure sensor 9 in the incoming section ABCDEFG of the loop-shaped main line sends a signal corresponding to the cryogenic agent pressure to the control and regulation system. The liquid phase sensor 10 sends a signal of complete filling of the loop-shaped main line (up to and including the bend 11) by the liquid cryogenic agent. Having received the signal from the sensor 10, the control and regulation system authorizes operating modes of the cryogenic apparatus. At the end of the mode for cryosurgical apparatus preparation for operation the feeding pump 6 is on, the shutoff valve 13 is closed, and the cryogenic agent is circulated in the loop-shaped main line.

In the mode 2, i.e. the operating mode for feeding cryogenic agent to the cryogenic instrument by generating pressure in the feeding cryoconduit for a closed-type cryogenic instrument, the feeding pump 6 is on, the shutoff valve 13 is open (as on the figure 1), and the cryogenic agent is fed to the closed-type cryogenic instrument via the feeding cryoconduit. The vacuum valve 22 serves to drain the contents of the gas-liquid phase draining cryoconduit to the air (see Figure 1). The vacuum pump 25 is off. The liquid cryogenic agent is fed to the cryogenic instrument 17 and evaporates to cool the cryogenic instrument. Gas phase is emitted to the air through the gas-liquid phase draining cryoconduit.

In the mode 3, i.e. the cryolavage mode or feeding the cryogenic agent to the cryogenic instrument by generating pressure in the feeding cryoconduit for an open-type cryogenic instrument with free discharge of cryogenic agent from the cryogenic instrument, wherein an open-type cryogenic instrument with free discharge of cryogenic agent (a spray nozzle 18) replaces the cryogenic instrument 17. In this case, the gas-liquid phase draining cryoconduit PQRS1 or PQRSTUV is not involved, and the vacuum pump is not activated.

In the mode 4, i.e. the mode for feeding the cryogenic agent to the cryogenic instrument by generating underpressure in the cryogenic instrument for a closed-type cryogenic instrument, the shutoff valve 13 is open, the cryogenic instrument 17 (of the closed-type) is connected to a cryosystem through the plug connection 16. The vacuum valve 22 connects the gas-liquid phase draining cryoconduit to the vacuum pump 25. The pressure controller 27 is set to zero. Once the vacuum pump is activated, an underpressure is generated inside the cryogenic instrument. Under such conditions, the liquid cryogenic agent is fed from the bend 11 to the cryogenic instrument 17, where it evaporates intensely to cool the surface of the cryogenic instrument.

In the mode 5, i.e. the mode for feeding cryogenic agent to the cryogenic instrument by generating underpressure in the cryogenic instrument for an open-type cryogenic instrument pressed to the tissues under treatment, the elements of the cryosurgical apparatus are in the same position as in the mode 5. Under such conditions, the liquid cryogenic agent is fed from the bend 11 to the cryogenic instrument 17 of the open-type, where it evaporates intensely to cool the surface of the cryogenic instrument.

In the mode 6, i.e. the warming mode, the first heater 14 is activated to evaporate and heat the cryogenic agent to the required temperature. The cryogenic instrument is warmed to the temperature, at which adhesion to the surface of the object being cooled is severed and the cryogenic instrument can be removed without damaging the object. In this mode, the valve 5 connects the gas phase intake 3 to the feeding pump 6. The gas phase cryogenic agent is fed to the cryogenic system and does not require evaporation by the heater 14, which serves to accelerate and enhance the warming of the cryogenic instrument.

A prototype model has been made in accordance with the invention. Tests of the prototype model demonstrated that this cryosurgical apparatus is more efficient in use as compared to the known art. The following results have been recorded:
1. Surgeries involving cryogenic instruments have proven to be more efficient due to the fact that the cryogenic agent (liquid nitrogen) does not boil inside the loop-shaped main line. Besides, the temperature of 210°C (boiling point of liquid nitrogen in vacuum) is maintained in the mode for cryogenic agent feeding by generating underpressure in the instrument.
2. Surgeries involving cryogenic instruments have proven to be more convenient due to the fact that the distance from the cryogenic instrument to the cryogenic agent containing reservoir can be increased to 2,5 meters.
3. Apparatus startup time is reduced as compared to the state-of-the-art devices that involve the cryogenic agent feeding directly from the reservoir under pressure.
4. It is possible to realize five operating modes of the cryosurgical apparatus, namely:
   I. Mode for feeding cryogenic agent to the cryogenic instrument by generating pressure in the feeding cryoconduit for a closed-type cryogenic instrument.
   II. Cryolavage mode or feeding cryogenic agent to the cryogenic instrument by generating pressure in the feeding cryoconduit for a spray nozzle with free discharge of the cryogenic agent from the cryogenic instrument.
   III. Mode for feeding cryogenic agent to the cryogenic instrument by generating underpressure in the cryogenic instrument for a closed-type cryogenic instrument.
   IV. Mode for feeding cryogenic agent to the cryogenic instrument by generating underpressure in the cryogenic instrument for an open-type cryogenic instrument pressed to the tissues under treatment.
   V. Warming mode - warming of a closed-type cryogenic instrument for safe removal of the same from the tissue under freezing.
5. It is possible to connect simultaneously several cryogenic instruments to the loop-shaped main line.
6. It is possible to connect a system for controlling and regulating the operation of the cryosurgical apparatus.

The proposed method for feeding cryogenic agent to the cryogenic instrument has been tested on the prototype model. Liquid nitrogen was used as the cryogenic agent. Tests of the prototype model in the mode for cryogenic agent feeding by generating underpressure in the cryogenic instrument showed an increase of the distance over which it is possible to feed the cryogenic agent to the cryogenic instrument from the reservoir with the cryogenic agent while keeping the cryogenic instrument at a low temperature. Using this method for feeding cryogenic agent to the cryogenic instrument, said distance between the cryogenic instrument and the reservoir was increased to 2,5 meters. At the same time the temperature of - 210°C (boiling point of liquid nitrogen in vacuum) was attained.

The cryosurgical apparatus proposed is recommended for use in healthcare institutions for cooling and destruction of pathologically changed areas of biological tissues.

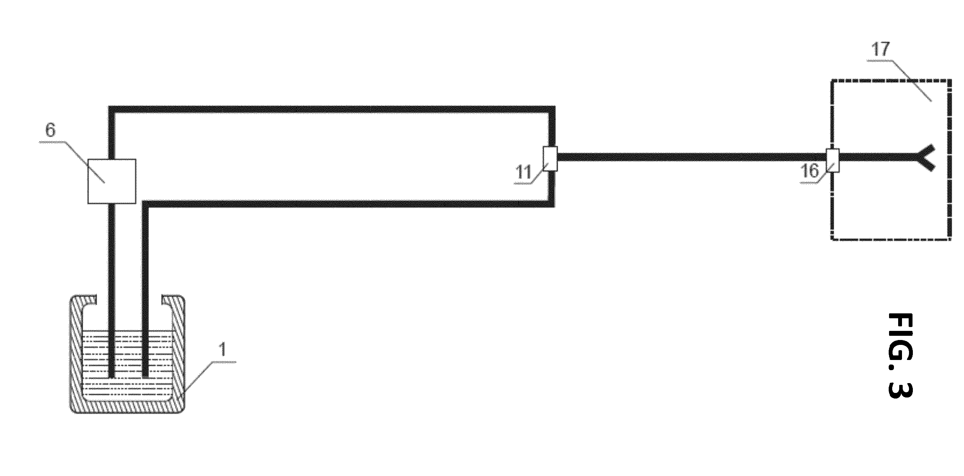

It should also be noted that it is relevant to add the pressure controlled 27 in the Figure 3 at the same location as in the Figure 1.

## Claims

1. Method for feeding a cryogenic agent to a cryogenic instrument, wherein one connects the cryogenic instrument to a reservoir with the cryogenic agent via a cryogenic conduit, feeds the cryogenic agent from the reservoir to the cryogenic instrument via the cryogenic conduit, ***characterized in that*** one forms a loop-shaped main line going out and entering into the reservoir with the cryogenic agent, realizes a circulation of the cryogenic agent into the loop-shaped main line, connects the cryogenic instrument to said loop-shaped main line via the cryogenic conduit and feeds through it the cryogenic agent, wherein a flow rate of the cryogenic agent circulated into the loop-shaped main line must be greater than a flow rate of the cryogenic agent entering into the cryogenic instrument via the cryogenic conduit.

2. Method for feeding a cryogenic agent to a cryogenic instrument according to the claim 1, ***characterized in that*** said feeding of the cryogenic agent from the loop-shaped main line to the cryogenic instrument is realized by generating an underpressure in the cryogenic instrument.

3. Method for feeding a cryogenic agent to a cryogenic instrument according to the claim 1, ***characterized in that*** said feeding of the cryogenic agent from the loop-shaped main line to the cryogenic instrument by generating a pressure in the cryogenic conduit connecting the loop-shaped main line and the cryogenic instrument.

4. Cryosurgical apparatus including a reservoir with a cryogenic agent, a cryogenic instrument with a feeding cryoconduit of the cryogenic agent and a draining cryoconduit of the cryogenic agent, an exhaust system of the cryogenic agent connected to the cryogenic instrument via the draining cryoconduit of the cryogenic agent, ***characterized in that*** the cryosurgical apparatus has a loop-shaped main line where circulates the cryogenic agent, an inlet and an outlet of the loop-shaped main line being connected to the reservoir with the cryogenic agent, said loop-shaped main line having at least one bend for connecting to the cryogenic instrument via the feeding cryoconduit of the cryogenic agent.

5. Cryosurgical apparatus according to the claim 4, ***characterized in that*** the inlet of the loop-shaped main line is connected to an outlet of an intake switch of a liquid or a gas phase of the cryogenic agent into the reservoir, while an inlet of the intake switch of the gas phase is connected to a section of the reservoir which contains the gas phase of the cryogenic agent and an inlet of the intake switch of the liquid phase is connected to a section of the reservoir which contains the liquid phase of the cryogenic agent.

6. Cryosurgical apparatus according to the claim 4, ***characterized in that*** the loop-shaped main line where circulates the cryogenic agent includes at least one feeding pump.

7. Cryosurgical apparatus according to the claim 4, ***characterized in that*** the loop-shaped main line where circulates the cryogenic agent includes at least one first controller of a cryogenic agent flow rate.

8. Cryosurgical apparatus according to the claim 4, ***characterized in that*** the loop-shaped main line where circulates the cryogenic agent includes at least one controller of a cryogenic agent pressure.

9. Cryosurgical apparatus according to the claim 4, ***characterized in that*** the loop-shaped main line where circulates the cryogenic agent includes at least one pressure sensor.

10. Cryosurgical apparatus according to the claim 4, ***characterized in that*** the loop-shaped main line where circulates the cryogenic agent includes at least one sensor of a cryogenic agent liquid phase in the closed main line.

11. Cryosurgical apparatus according to the claim 4, ***characterized in that*** the feeding cryoconduit of the cryogenic agent from the loop-shaped main line to the cryogenic instrument includes at least one clack valve of the cryoconduit.

12. Cryosurgical apparatus according to the claim 4, ***characterized in that*** the feeding cryoconduit of the cryogenic agent from the loop-shaped main line to the cryogenic instrument includes at least one shutoff valve.

13. Cryosurgical apparatus according to the claim 4, ***characterized in that*** the feeding cryoconduit of the cryogenic agent from the loop-shaped main line to the cryogenic instrument includes at least one first heater of the cryogenic agent.

14. Cryosurgical apparatus according to the claim 4, ***characterized in that*** the feeding cryoconduit of the cryogenic agent from the loop-shaped main line to the cryogenic instrument includes at least one first sensor of temperature of the cryogenic agent.

15. Cryosurgical apparatus according to the claim 4, ***characterized in that*** the feeding cryoconduit of the cryogenic agent from the loop-shaped main line to the cryogenic instrument includes at least one plug connection for connecting various cryogenic instruments.
